# EUROPEAN PATENT SPECIFICATION

(11) **EP 1 590 353 B1**
(45) Date of publication and mention of the grant of the patent: **10.10.2007**
(21) Application number: 02808277.4
(22) Date of filing: 20.12.2002
(51) Int. Cl.: C07D 501/00

(54) **A PROCESS FOR THE PREPARATION OF CEFPODOXIME PROXETIL**
VERFAHREN ZUR HERSTELLUNG VON CEFPODOXIM-PROXETIL
PROCEDE POUR PREPARER DU CEFPODOXIME PROXETIL

(43) Date of publication of application: 02.11.2005
(73) Proprietor: Lupin Limited, Mumbai 400 098, Maharashtra (IN)
(72) Inventor: GHARPURE, Milind, M. Emcure Pharmaceutical Limited, Pune 411 018 (IN); DESHMUKH, Sanjay, S. Lupin Limited Survey 46/47A, Pune 411 042 Maharashtra (IN); SAHA, Asok, K. Lupin Limited Survey No. 46A/47A, Pune 411 042 Maharashtra (IN); MAHALE, Rajendra, D. Lupin Limited Survey 46/47A, Pune 411 042 Maharashtra (IN)
(74) Representative: Jennings, Nigel Robin
(86) International application number: PCT/IN2002/000240
(87) International publication number: WO 2004/056834

(56) References cited:
- WO-A-01/34611
- WO-A-02/068429
- US-A- 4 400 503

## Description

### Field of Invention

The present invention provides a simple industrial method for obtaining cefpodoxime proxetil of high purity conforming to pharmaceutical specifications.

### Background of the invention

Cefpodoxime Proxetil of formula (I), chemically known as 1-isopropoxycarbonyloxyethyl(6R,7R)-7-[2-(2-aminothiazol-4-yl)-2(Z)-(methoxyimino)acetamido]-3-(methoxymethyl)-3-cephem-4-carboxylate belongs to the third generation of cephalosporin antibiotics, which is administered orally.

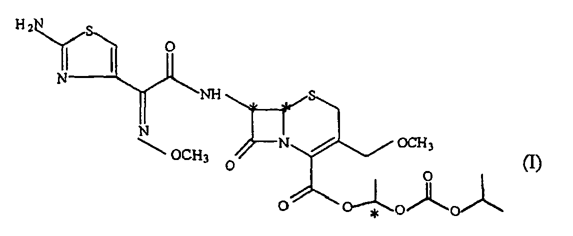

Cefpodoxime proxetil of formula (I) has two asymmetric centers at position 6 and 7 of the cephem nucleus and another one at the α-carbon of 1-isopropoxycarbonyloxyethyl group attached to the 4-carboxyl group as shown in the above structure. The asymmetric center of the α-carbon of 1-isopropoxycarbonyloxyethyl group attached to the 4-carboxyl group of Cefpodoxime proxetil of formula (I) exists as a pair of diastereoisomers known as the R and S isomer. Pharmacopoeial forum Vol: 28 (1), pp 44-52, (2002) mentions that the diastereomeric ratio (R / R+S) of cefpodoxime proxetil should be between 0.5 to 0.6, where the R isomer is less polar among the two stereoisomers.

US Patent No. 4, 486, 425 (Nakao, et al) discloses several methods for preparation of cefpodoxime proxetil of formula (I). However, all the methods are associated with formation of varying amounts of impurities. The associated impurities have been removed by taking recourse to chromatographic separation techniques, which albeit provides the compound of formula (I) of desired quality, but is however, cumbersome and not practical on industrial scale.

The impurities which are normally associated with manufacturing processes for cefpodoxime proxetil to name a few, are the Δ² isomer and the E-isomer of cefpodoxime proxetil which have the chemical structures shown below:

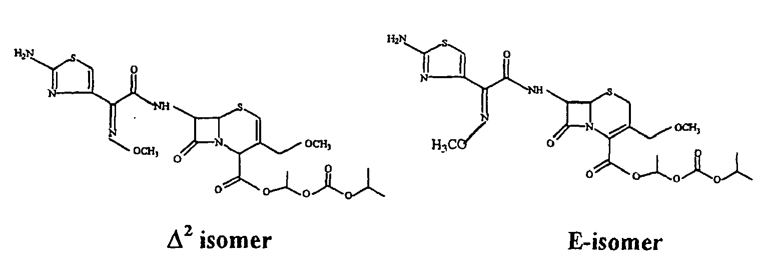

Journal of Antibiotics (Vol: 40, 370, 1987; Fujimoto. K; et. al) describes another method for preparation of cefpodoxime proxetil which consists of the conversion of 7-[2-(2-chloroacetyl amino thiazol-4-yl)-2-(2-methoxyimino acetamido]-3-acetoxy-methyl-3-cephem-4-carboxylic acid (V) into the corresponding 3-methoxymethyl derivative, by reaction with methanol and CaCl₂.2H₂O, which on deprotection of the amino protecting group at the 2-aminothiazolyl ring gives cefpodoxime acid (VI).

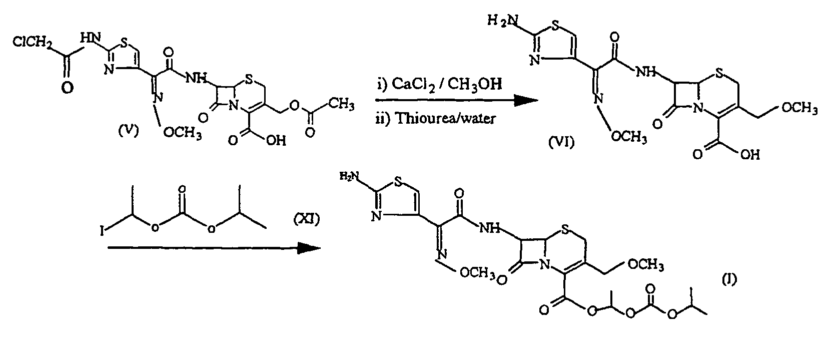

Esterification of the carboxylic acid with 1-iodoethylisopropyl carbonate gives the compound of formula (I).

However, this method also takes recourse to chromatographic methods for obtaining the product in pure form. Moreover, this method also involves additional steps of protection and deprotection of the amino group resulting in overall lower efficiency.

PCT Appln WO 99/35149,A1 (Greil et.al) describes yet another method for preparation of cefpodoxime proxetil of formula (I), wherein, Z-2-(methoxyimino)-2-(2-formylaminothiazol-4-yl) acetic acid (VII) activated as the acid chloride hydrochloride on reaction with a compound of formula (VIII), gives N-formylcefpodoxime proxetil of formula (IX).

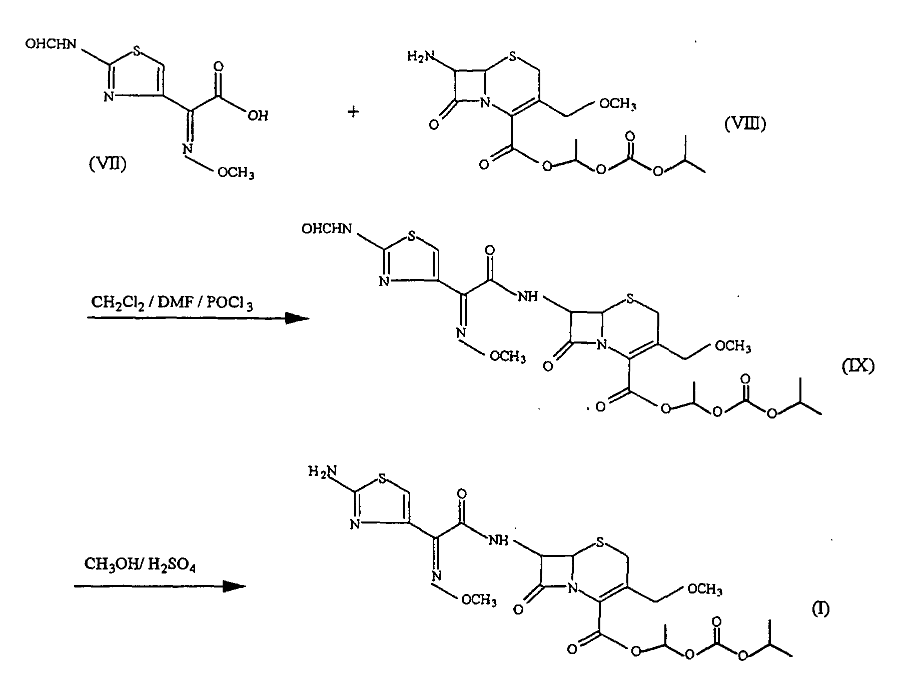

In this method, the intermediate compound of formula (IX) is purified by treating it with water / alcohol mixture containing an additive, such as an organic amide, urea, an imidazolidinone or a pyrimidinone. The purified compound (IX) thus obtained on treatment with acid gives cefpodoxime proxetil of formula (I).

Even though this method does not involve purification, utilizing column chromatography in the final step, additional steps of protection of the amino group as the formyl derivative and its subsequent deprotection are required.

US Patent Appln No.2002/0065262 A1 (Greil. J, et al) describes a method for preparation of cefpodoxime proxetil having the right diastereomeric ratio and chemical purity via the intermediacy of a crystalline form of 7-[2-(2-formylaminothiazol-4-yl)-2-(Z)-(methoxyimino)acetamido]-3-methoxymethyl-3-cephem-4-carboxylic acid-1-(isopropoxycarbonyloxy) ethyl ester of formula (IX).

The preparation of the crystalline form of n-formyl cefpodoxime proxetil of formula (IX) comprises reaction of 7-amino-3-methoxymethyl-3-cephem-4-carboxylic acid (X) with 1-iodoethyl isopropyl carbonate (XI) to give the compound (VIII), which on condensation with Z-(2-formylaminothiazo-4-yl)-methoxyimino acetic acid activated as an ester or a halogenide gives N-formyl cefpodoxime proxetil which is then crystallized from a mixture of a nitrile or a ketone with water to get crystalline N-formyl cefpodoxime of formula (IX) having a diastereomeric ratio between 0.5 to 0.6.

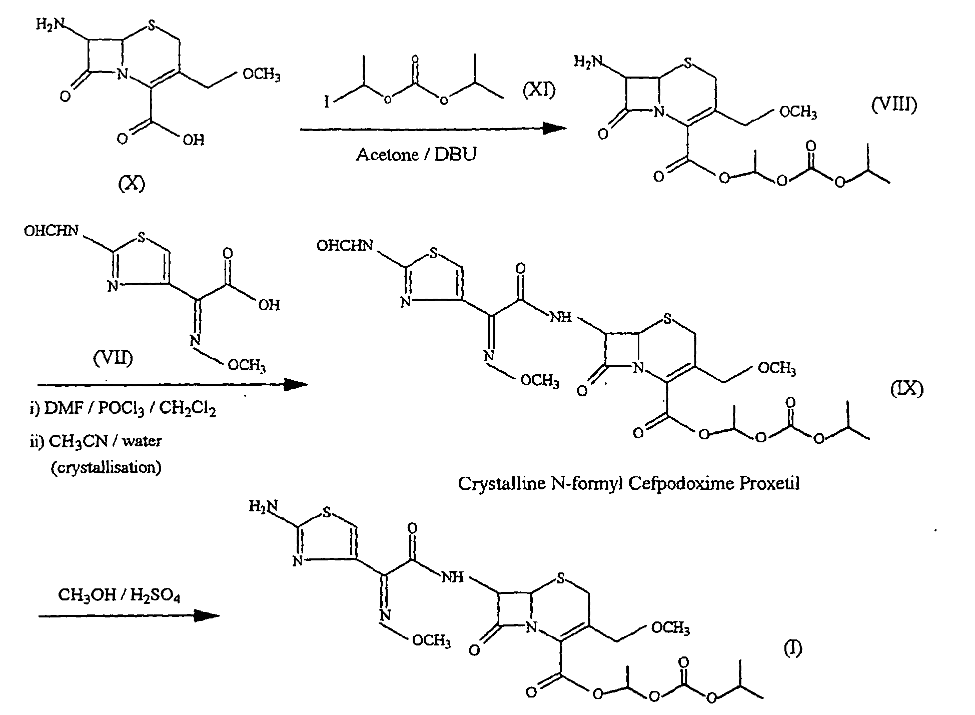

The compound (IX) is then treated with H₂SO₄/methanol to get cefpodoxime proxetil having isomeric ratio between, 0.5 to 0.6.

This method also requires a protection and a deprotection step in addition to a crystallisation step, to give cefpodoxime proxetil, which decreases the overall yield.

PCT Appln WO 02068429 A1 (Yatendra Kumar, et al) discloses a method for purification and isolation of cefpodoxime proxetil, obtained by reacting cefpodoxime acid of formula (VI), with 1-iodoethyl isopropylcarbonate of formula (XI) in the presence of a base.

The purification of impure cefpodoxime proxetil thus obtained is carried out in two stages which comprises of:
i) dissolving impure cefpodoxime proxetil or adding a solution containing cefpodoxime proxetil into a polar organic solvent or mixture(s) thereof, optionally reducing the solvent by concentration, and adding into a non-polar organic solvent or mixture(s) thereof to precipitate the solid; and
ii) dissolving the solid obtained from the above step into a water miscible polar organic solvent, optionally reducing the solvent by concentration, and adding into water to obtain the pure cefpodoxime proxetil.

However, this method involves a two-step isolation and purification, which moreover involves use of a combination of solvents for crystallization, rendering the method tedious and not commercially attractive.

To summarize, cefpodoxime proxetil prepared by various methods as mentioned herein earlier, is invariably contaminated with varying amounts of impurities associated with the respective method of preparation.

The methods employed for purification of cefpodoxime proxetil utilizes,
i) separation techniques like chromatography,
ii) protection , deprotection of reactive functional groups,
iii) multi-step crystallization,
iv) use of combination of solvents for crystallization,
which renders such methods less suitable for industrial application.

Therefore, a need exists for a simple, cost-effective method for obtaining cefpodoxime proxetil of high purity, which overcomes the shortcomings of the prior art methods. Such a need has been met by the present invention, which is described in detail herein below.

### OBJECTS OF THE INVENTION

It is an object of the present invention to provide a method for preparation of cefpodoxime proxetil of high purity.

Another object of the present invention is to provide a method for preparation of cefpodoxime proxetil of high purity, which is simple and cost-effective.

Yet another object of the present invention is obtain cefpodoxime proxetil of high purity comprising formation of an acid addition salt of impure cefpodoxime proxetil and its crystallization from an organic solvent.

Yet, further object relates to the preparation of the acid addition salt, which is free from impurities.

A further object relates to the preparation of cefpodoxime proxetil of high-purity and conforming to pharmacopoeial specification from cefpodoxime proxetil acid addition salt comprising neutralization of the acid addition salt by conventional methods.

### SUMMARY OF THE INVENTION

The above and the other objects of the present invention are achieved by the process of the present invention which comprises:
a) adding hydrogen halide to a solution of impure cefpodoxime proxetil in methyl isobutyl ketone and isolating the hydrohalide salt of cefpodoxime proxetil thus formed, and 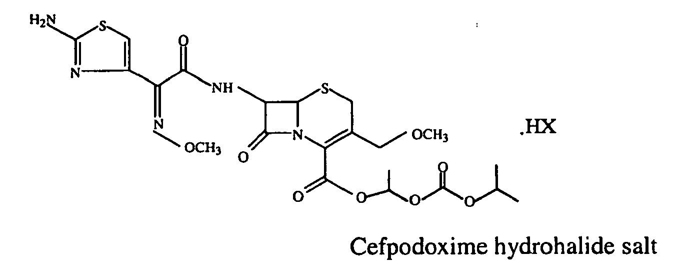
b) dissolving the cefpodoxime proxetil hydrohalide salt obtained in the above step in a water-miscible or water-immiscible organic solvent and neutralizing the salt thus formed with a base followed by isolation of cefpodoxime proxetil in pure form.

In an embodiment of the invention, said water miscible organic solvent in step b) is selected from an alcohol, tetrahydrofuran and acetonitrile.

The alcohol is preferably selected from methanol, ethanol, n-propanol, isopropanol, n-butanol, isobutyl alcohol, tertiary butanol.

In a preferred embodiment, said water immiscible solvent in step b) is selected from a ketonic solvent, ethyl acetate, methyl isobutyl ketone, chloroform, dichloromethane and 1,2-dichloroethane.

In an embodiment of the invention, the methyl isobutyl ketone is employed in a volume of from 2.0 to 7.0 times the weight of the impure cefpodoxime proxetil.

The hydrohalide is selected preferably hydrochloric acid, hydrobromic acid and hydroiodic acid.

In a preferred embodiment, the molar ratio of the hydrogen halide used is 1.0 to 1.5 times of cefpodoxime proxetil.

In an embodiment of the invention, said hydrohalide salt is isolated by filtration.

In a preferred embodiment, said pure cefpodoxime proxetil has a diastereomeric ratio between 0.50 and 0.60.

### DETAILED DESCRIPTION OF THE INVENTION

The method for preparation of cefpodoxime proxetil of high purity as per the present invention comprises of the following steps; as summarized in scheme-I.

The first step consists of reaction of cefpodoxime acid of formula (VI) with 1-iodoethyl isopropylcarbonate of formula (XI), to give cefpodoxime proxetil of formula (I).

### Synthesis of cefpodoxime proxetil of formula (I):

Typically, cefpodoxime acid of formula (VI) (prepared by any of the general methods reported in US Patent No. 4, 486, 425) is dissolved in any of the solvents like N,N-dimethyl acetamide, N,N-dimethyl formamide, dimethyl sulphoxide but preferably N,N-dimethyl acetamide which is in a ratio of 5-10 times volume per gram of cefpodoxime acid taken.

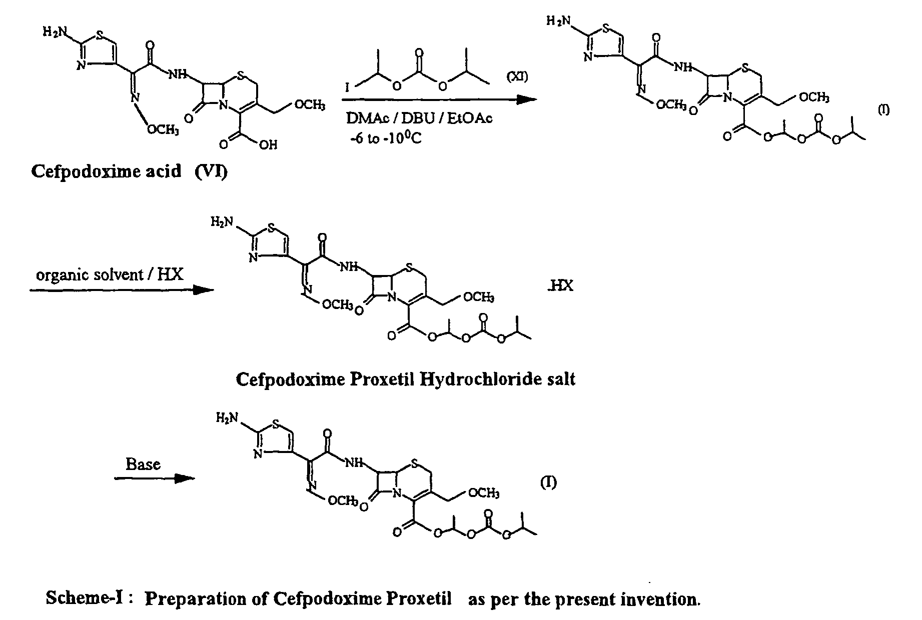

The acid is converted to its salt by using a base selected from a group of inorganic bases such as potassium carbonate, sodium carbonate and sodium bicarbonate preferably sodium carbonate, or from a group of organic bases consisting of dicyclohexyl amine, 1,8-diazabicyclo [5,4,0] undec-7-ene (DBU) preferably the latter, or a mixture thereof.

The molar ratio of 1,8-diazabicyclo [5,4,0] undec-7-ene (DBU) or sodium carbonate used, being in the range of 0.95 to 1.02 mole equivalent, but preferably 0.96 to 0.98 mole equivalent with respect to the acid used.

The reaction temperature employed during the salt formation should be between -20° to 0°C, but preferably between -5°C to -10°C.

1-Iodoethylisopropyl carbonate of formula (XI) is then added to the salt of the carboxylic acid at a temperature ranging between -20°C to 0°C, but preferably between -5°C to -10°C. The molar ratio of 1-Iodoethylisopropyl carbonate used being in the range of 0.96 to 1.20 moles.

The duration of addition of 1-iodoethylisopropyl carbonate of formula (XI) can be between 5 to 30 minutes but preferably between 5 to 15 minutes.

The reaction temperature employed can be between -20°C to 0°C, but preferably between -5°C to -10°C, and the reaction time between 10 to 60 minutes but preferably between 20 to 30 minutes.

Reaction mixture is quenched with a dilute inorganic acid like sulphuric acid, hydrochloric acid but preferably dilute hydrochloric acid.

The quenched reaction mixture is then extracted with an organic solvent like alkyl acetates, which includes methyl acetate, ethyl acetate, and butyl acetate but preferably ethyl acetate.

Organic layer is washed with dilute alkali carbonate solution of alkali carbonates like sodium carbonate, sodium bicarbonate, potassium carbonate but preferably a dilute solution of sodium carbonate. The temperature during the alkali wash should be in the range of -5°C to +5°C but preferably between 0°C to 5°C.

Organic layer is subsequently washed with 5% sodium thiosulphate solution.

Organic layer is optionally treated with carbon and filtered. The filtrate is evaporated under reduced pressure to give an oily residue.

The temperature during the evaporation of the organic solvent can be in the range of 15-35°C as higher temperature is detrimental for the purity of the compound.

In a specific embodiment, a clear solution of cefpodoxime acid (VI) in dimethyl acetamide is cooled to -5°C and 1,8-diazabicyclo[5,4,0] undec-7-ene (DBU) in stoichiometric amounts is added in 10 minutes at the same temperature. The reaction mixture is agitated for 5-10 minutes after addition, for complete salt formation after which 1-iodoethylisopropyl carbonate (XI) is added at -6°C to -10°C in 10 minutes.

The reaction mixture is agitated for 20 to 30 minutes at -6 °C to -8°C, quenched with 10% hydrochloric acid and the aqueous layer successively extracted with ethyl acetate.

Organic layer is agitated with 2% sodium carbonate solution, at 0-5°C for 30 minutes and then washed with 5% sodium thiosulphate solution.

Organic layer is optionally treated with activated carbon and filtered. The filtrate is concentrated at reduced pressure and at a temperature not exceeding 35°C to give a brownish oily residue.

### Purification of cefpodoxime proxetil (I): Formation of acid addition salt.

The next step comprises of preparation of the acid addition salt of cefpodoxime proxetil by treatment of cefpodoxime proxetil dissolved in a suitable organic solvent with an inorganic acid.

The acid addition salt formation is carried out in a water immiscible organic solvent, namely methyl isobutyl ketone. This is used because the acid addition salt crystallizes out easily and moreover the diastereomeric ratio of the R and S isomer is maintained within permissible limits and also the associated impurities are reduced.

The volume of the methyl isobutyl ketone used is between 2.0 to 7.0 times the weight of impure Cefpodoxime proxetil obtained.

For the acid addition salt formation, hydrohalic acids are used since such salts crystallize out easily. The hydrohalic acids that can be used include hydrochloric acid, hydrobromic acid and hydroiodic acid but hydrochloric acid is preferred.

The hydrohalide salts can be prepared by adding an aqueous solution of the corresponding hydrohalic acid to a solution of impure Cefpodoxime proxetil in the immiscible organic solvent. The salts can also be prepared by bubbling the respective hydrohalide gas into a solution of impure cefpodoxime proxetil in the ketonic solvent.

The molar range of the acid used, is in the range of 1.0 to 1.2.moles per mole of cefpodoxime proxetil.

The acid addition salt formation can be carried out in the temperature range of 0 to 40°C, but preferably between 25°C to 30°C.

The time required for formation of the acid addition salt can be in the range of 1 to 6 hours, but preferably 2 to 5 hours.

In a specific embodiment, the impure cefpodoxime proxetil of formula (I), obtained in the first part of the synthesis is diluted with methyl isobutyl ketone and stirred at 25°C to completely dissolve the residue.

Concentrated hydrochloric acid is added in 5-10 minutes and the resulting mixture is stirred for 2.0 hours for complete precipitation of cefpodoxime proxetil hydrochloride salt. Filtration of the hydrochloride salt gives cefpodoxime proxetil hydrochloride, which can be dried or used as such without drying, for the next step.

Alternatively, the reaction mixture in the first step can be extracted with methyl isobutyl ketone, and after washing with water and sodium thiosulphate solution, the organic layer can be used as such for hydrohalide salt formation, with hydrohalic acid. The volume of the ketonic solvent in the extract is brought in the range of 2.0 to 7.0 times the weight of the impure cefpodoxime proxetil by partial evaporation of the solvent.

The diastereomeric ratio of the R & S isomer present in the hydrohalide salt is in the range of 0.50 to 0.60 which conforms to the specified range of 0.5 to 0.6 mentioned in Pharmacopoeial Forum Vol. 28 (1), pp 44-52, (2002).

The hydrochloride salt is found to be relatively free from all associated impurities and most important the ratio of the R and S isomer is found to remain intact.

### Neutralisation of cefpodoxime proxetil hydrohalide salt.

The next step comprises of neutralizing cefpodoxime proxetil hydrochloride with a base in a water miscible or water immiscible solvent and isolating cefpodoxime proxetil of high purity, which conforms to pharmacopoeial specification.

The cefpodoxime proxetil hydrohalide salt either dried or wet as obtained in the second step can be dissolved in a water miscible solvent and mixed with an aqueous solution of a base. Pure cefpodoxime proxetil is isolated by addition of water. The water miscible solvents include alcoholic solvents, tetrahydrofuran and the nitriles. The alcoholic solvents includes ethanol, methanol but preferably methanol and the nitriles include acetonitrile.

Alternatively, the compound is dissolved in a water immiscible solvent like ethyl acetate, methyl isobutyl ketone, chloroform, dichloromethane, 1,2-dichloroethane but preferably ethyl acetate, and agitated with the solution of a mild base, for complete neutralization of the hydrohalide salt. The organic layer is separated and the solvent can be concentrated to give pure cefpodoxime proxetil.

The base used for liberating the free base viz. cefpodoxime proxetil from its hydrochloride salt can be an inorganic base. The inorganic bases used include sodium hydroxide, sodium carbonate, sodium bicarbonate, potassium bicarbonate; Mild bases are preferred and among them, the alkali bicarbonates, which include sodium bicarbonate and potassium bicarbonate, are preferred, as impurity formation is minimised. Among them, sodium bicarbonate is preferred, as it is milder as compared to potassium bicarbonate.

The time required for addition of the mixture containing cefpodoxime proxetil hydrohalide salt to the alkaline solution varies between 15 to 45 minutes but preferably 30 minutes.

The temperature range is between, 15°C to 40°C but the preferred range is 25°C to 30°C.

In a specific embodiment, cefpodoxime proxetil hydrochloride is dissolved in methanol, at 25°C to 30°C and is added to a 1% solution of sodium bicarbonate. The reaction mixture is agitated for 60 minutes. The cefpodoxime proxetil separating out is filtered and dried.

The diastereomeric ratio of the R and S isomer is between, 0.50 to 0.60, which conforms to pharmacopoeial specification.

The invention can be further illustrated by the following examples, which, however, should not be construed as limiting the scope of the invention.

### Example-1

### Preparation of Cefpodoxime Proxetil (I) :

Cefpodoxime acid of formula (VI) (50gms; 0.117moles) was added to dimethyl acetamide (350ml) and stirred to get a clear mixture. The mixture was cooled to -6 to -10 °C. 1,8-diazabicyclo [5,4,0] undec-7-ene (DBU) (17.4gms; 0.114moles) was added isopropyl carbonate (30.18gms; 0.117moles) was then added slowly to the reaction mixture in a period of 10 to 15 minutes. The reaction mixture was agitated for a period of 20 to 30 minutes at the same temperature. The reaction mixture was quenched by addition of 13% hydrochloric acid. The reaction mixture was further diluted with water (400 ml) and extracted with ethyl acetate (500ml). The separated aqueous layer was re-extracted with ethyl acetate (500ml). The organic layers were combined and stirred with 2% sodium carbonate solution (500ml) at 0 to 5°C for 30 minutes. The organic layer was washed with 5% sodium thiosulphate solution (500 ml). Organic layer was treated with charcoal (7.5gms) at 25°C for 60 minutes and filtered through celite bed. The filtrate was concentrated at reduced pressure and at a temperature, below 35°C. The residue was carried forward as such to the next step.
Isomer ratio: (R/R+S): 0.51

### Example 2:

### a) Preparation of Cefpodoxime Proxetil hydrochloride using methyl isobutyl ketone as solvent.

Impure cefpodoxime proxetil (10gms; 0.018moles) as obtained in example-1 was dissolved in methyl isobutyl ketone (30ml) and stirred at 25°C. Concentrated hydrochloric acid (1.8gms; 0.021moles) was added to the mixture in 5 minutes and stirred for 120 minutes at the same temperature. The reaction mixture was filtered to give cefpodoxime proxetil hydrochloride (9.6 gms, 90% yield).
Isomer ratio: (R/R+S): 0.52

### b) (Comparative Example) Preparation of Cefpodoxime Proxetil hydrochloride using acetone as solvent

Impure cefpodoxime proxetil (16.0gms; 0.029moles) as obtained in example-1 was dissolved in acetone (32ml) and stirred at 25°C. Concentrated hydrochloric acid (3.6gms; 0.034moles) was added to the mixture in 5 minutes and stirred for 120 minutes at the same temperature. The reaction mixture was filtered to give cefpodoxime proxetil hydrochloride (11.2gms; 66% yield).
Isomer ratio: (R/R+S): 0.54.

### Example 3:

### Preparation of Cefpodoxime Proxetil hydrobromide using methylisobutyl ketone as solvent

Cefpodoxime proxetil (5gms; 0.00897) was dissolved in methyl isobutyl ketone (15ml) and stirred at 25°C. A 49% solution of hydrobromic acid (1.7gms; 0.011moles) was added to the mixture in 5 minutes and stirred for 300 minutes at the same temperature. Cooled to -10°C and stirred for 1.0 hr. The reaction mixture was filtered to give cefpodoxime proxetil hydrobromide (4.5gms, 79% yield). Isomer ratio: (R/R+S): 0.586

### Example 4:

### a) Preparation of pure Cefpodoxime proxetil from Cefpodoxime proxetil hydrochloride salt using methanol as solvent

Cefpodoxime proxetil hydrochloride (9.6gms; 0.016moles) obtained in example 2a or 2b or 2c was dissolved in methanol (38ml) at 25-30°C and added to a solution of sodium bicarbonate (1.5gms; 0.016moles) in water (160ml) in 30 minutes at 25 to 28°C. The mixture was stirred for 60 minutes. The product separating out was filtered and washed with water 2 x 25ml and dried to give pure cefpodoxime proxetil (Yield: 7 gms; %yield: 77.7%; diastereomeric ratio; (R/R+S):0.54).
Purity: ≥ 95 %.

### b) Preparation of pure Cefpodoxime proxetil from Cefpodoxime proxetil hydrobromide salt using methanol as solvent.

Cefpodoxime proxetil hydrobromide (10gms; 0.016moles) obtained in example 3 was dissolved in methanol (228ml) at 25-30°C and added to a solution of sodium bicarbonate (1.55gms; 0.018moles) in water (160ml) in 30 minutes at 25 to 28°C. The mixture was stirred for 60 minutes. The product separating out was filtered and washed with water 2 x 25ml and dried to furnish pure cefpodoxime proxetil (6.50gms, 75.1%yield; diastereomeric ratio; {R/R+S}: 0.59).
Purity: ≥ 95%.

### c) Preparation of pure Cefpodoxime proxetil from Cefpodoxime hydrochloride salt using acetonitrile as solvent

Cefpodoxime proxetil hydrochloride (3.0gms; 0.00538moles) obtained in example 2a or 2b or 2c or was dissolved in acetonitrile (21ml) at 25-30°C and added to a solution of sodium bicarbonate (0.55gms; 0.00654moles) in water (375ml) in 30 minutes at 25 to 28°C. The mixture was stirred for 60 minutes. The product separating out was filtered and washed with water 2 x 25ml and dried to furnish pure cefpodoxime proxetil (1.8gms, 63.9 % yield; diastereomeric ratio; {R/R+S} 0.54); Purity: 92.6%

### d) Preparation of pure Cefpodoxime proxetil from Cefpodoxime hydrochloride using tetrahydrofuran as solvent

Cefpodoxime proxetil hydrochloride (3.0gms; 0.0054moles) obtained in example 2a or 2b or 2c or was dissolved in tetrahydrofuran (24ml) at 25-30°C and added to a solution of sodium bicarbonate (0.55gms; 0.0065moles) in water (375ml) in 30 minutes at 25 to 28°C. The mixture was stirred for 60 minutes. The product separating out was filtered and washed with water 2 x 25ml and dried to furnish pure cefpodoxime proxetil (1.2 gms, 42.6 % yield; diastereomeric ratio; {R/R+S}: 0.54).
Purity: ≥ 92%

## Claims

1. A process for the preparation of cefpodoxime proxetil of formula (I), of high purity conforming to pharmacopoeial specifications, 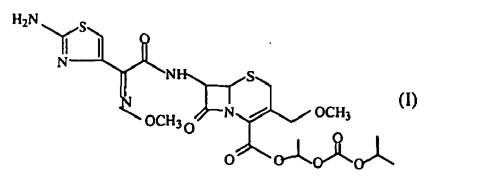 which comprises
a) adding hydrogen halide to a solution of impure cefpodoxime proxetil in methyl isobutyl ketone and isolating the hydrohalide salt of cefpodoxime proxetil thus formed, and 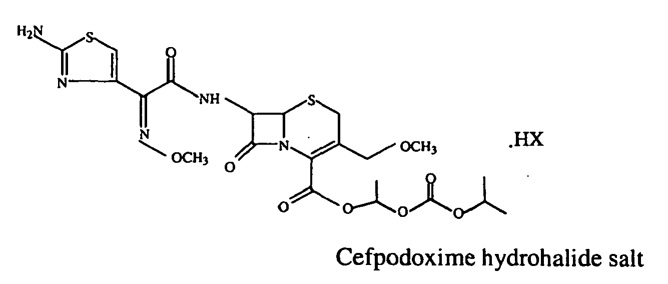
b) dissolving the cefpodoxime proxetil hydrohalide salt obtained in the above step (a) in a water-miscible or water-immiscible organic solvent and neutralizing the salt thus formed with a base followed by isolation of cefpodoxime proxetil in pure form.

2. A process according to claim 1, wherein said water miscible organic solvent in step (b) is selected from an alcohol, tetrahydrofuran and acetonitrile.

3. A process according to claim 2, wherein said alcohol is selected from methanol, ethanol, n-propanol, isopropanol, n-butanol, isobutyl alcohol, tertiary butanol.

4. A process according to claim 1 wherein said water immiscible solvent in step (b) is selected from ethyl acetate, methyl isobutyl ketone, chloroform, dichloromethane and 1,2-dichloroethane.

5. A process according to any preceeding claims, wherein the methyl isobutyl ketone is employed in a volume of from 2.0 to 7.0 times the weight of the impure cefpodoxime proxetil.

6. A process according to any preceding claim , wherein said hydrohalide is selected from hydrochloric acid, hydrobromic acid and hydroiodic acid.

7. A process according to claim 6 , wherein the molar ratio of the hydrogen halide used is 1.0 to 1.5 times of cefpodoxime proxetil.

8. A process according to of claim 1, wherein the hydrohalide salt is isolated by filtration.

9. A process according to any preceding claim, wherein said base is an inorganic base.

10. A process according to claim 9, wherein said inorganic base is selected from sodium bicarbonate, sodium hydroxide, sodium carbonate, potassium carbonate and potassium bicarbonate.

11. A process according to any preceding claim, wherein the pure cefpodoxime proxetil is isolated by filtration.

12. A process according any preceding claim, wherein said pure cefpodoxime proxetil has a diastereomeric ratio between 0.50 and 0.60.

13. A process as claimed in any preceding claim wherein said treatment of hydrohalide salt of cefpodoxime with said base is carried out in 15 to 45 minutes, preferably, 30 minutes.

14. A process as claimed in claim 1 or 13 wherein said treatment with said base is carried out at a temperature of 15 to 40°C., preferably, 25 to 30°C.

15. A process as claimed in any preceding claim wherein after said treatment with said base, said reaction mixture is agitated for 60 minutes.

## Patentansprüche

1. Verfahren zur Herstellung von Cefpodoximproxetil der Formel (I) von hoher Reinheit entsprechend Pharmakopöe-Vorschriften, 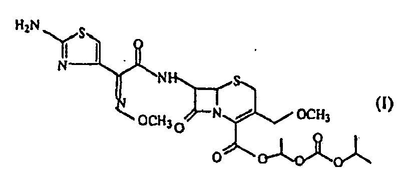 welches umfasst:
a) Zugeben von Halogenwasserstoff zu einer Lösung aus unreinem Cefpodoximproxetil in Methylisobutylketon und Isolieren des so gebildeten Halogenwasserstoffsalzes von Cefpodoximproxetil und 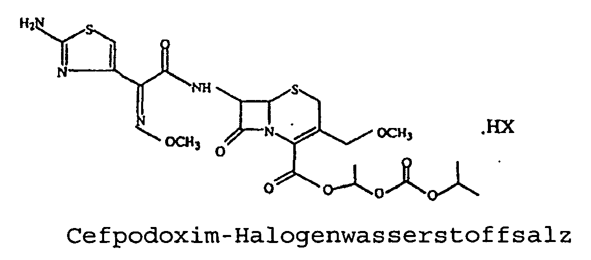
b) Lösen des im obigen Schritt (a) erhaltenen Cefpodoximproxetil-Halogenwasserstoffsalzes in einem mit Wasser mischbaren oder mit Wasser nicht mischbaren organischen Lösungsmittel und Neutralisieren des so gebildeten Salzes mit einer Base, gefolgt von Isolierung von Cefpodoximproxetil in reiner Form.

2. Verfahren gemäß Anspruch 1, worin besagtes mit Wasser mischbares organisches Lösungsmittel in Schritt (b) ausgewählt wird aus einem Alkohol, Tetrahydrofuran und Acetonitril.

3. Verfahren gemäß Anspruch 2, worin besagter Alkohol ausgewählt wird aus Methanol, Ethanol, n-Propanol, Isopropanol, n-Butanol, Isobutylalkohol, tert.-Butanol.

4. Verfahren gemäß Anspruch 1, worin besagtes mit Wasser nicht mischbares Lösungsmittel in Schritt (b) ausgewählt wird aus Ethylacetat, Methylisobutylketon, Chloroform, Dichlormethan und 1,2-Dichlorethan.

5. Verfahren gemäß einem vorhergehenden Anspruch, worin das Methylisobutylketon in einem Volumen von 2,0- bis 7,0-mal dem Gewicht des unreinen Cefpodoximproxetil eingesetzt wird.

6. Verfahren gemäß einem vorhergehenden Anspruch, worin besagter Halogenwasserstoff ausgewählt wird aus Salzsäure, Bromwasserstoffsäure und Iodwasserstoffsäure.

7. Verfahren gemäß Anspruch 6, worin das Molverhältnis des verwendeten Halogenwasserstoffs 1,0- bis 1,5-mal von Cefpodoximproxetil ist.

8. Verfahren gemäß Anspruch 1, worin das Halogenwasserstoffsalz durch Filtration isoliert wird.

9. Verfahren gemäß einem vorhergehenden Anspruch, worin besagte Base eine anorganische Base ist.

10. Verfahren gemäß Anspruch 9, worin besagte anorganische Base ausgewählt wird aus Natriumbicarbonat, Natriumhydroxid, Natriumcarbonat, Kaliumcarbonat und Kaliumbicarbonat.

11. Verfahren gemäß einem vorhergehenden Anspruch, worin das reine Cefpodoximproxetil durch Filtration isoliert wird.

12. Verfahren gemäß einem vorhergehenden Anspruch, worin besagtes reines Cefpodoximproxetil ein diastereomeres Verhältnis zwischen 0,50 und 0,60 hat.

13. Verfahren wie in einem vorhergehenden Anspruch beansprucht, worin besagte Behandlung von Halogenwasserstoffsalz von Cefpodoxim mit besagter Base in 15 bis 45 Minuten, vorzugsweise 30 Minuten durchgeführt wird.

14. Verfahren wie in Anspruch 1 oder 13 beansprucht, worin besagte Behandlung mit besagter Base bei einer Temperatur von 15 bis 40°C, vorzugsweise 25 bis 30°C durchgeführt wird.

15. Verfahren wie in einem vorhergehenden Anspruch beansprucht, worin nach besagter Behandlung mit besagter Base besagtes Umsetzungsgemisch für 60 Minuten bewegt wird.

## Revendications

1. Procédé pour la préparation de cefpodoxime proxétil de formule (I), d'une grande pureté répondant aux spécifications de la pharmacopée, 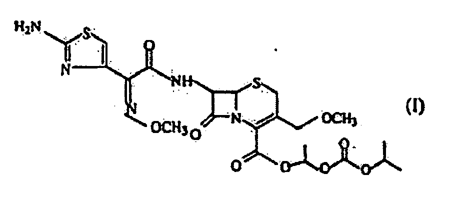 qui comprend :
a) l'addition d'halogénure d'hydrogène à une solution de cefpodoxime proxétil impur dans de la méthylisobutylcétone et l'isolement du sel d'halogénohydrate de cefpodoxime proxétil ainsi formé et 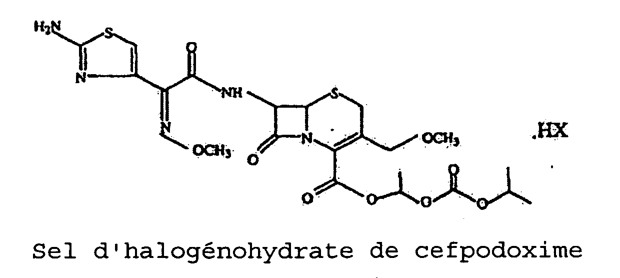
b) la dissolution du sel d'halogénohydrate de cefpodoxime proxétil obtenu à l'étape (a) décrite ci-dessus dans un solvant organique miscible ou non miscible à l'eau et la neutralisation du sel ainsi formé avec une base suivie par l'isolement du cefpodoxime proxétil sous forme pure.

2. Procédé selon la revendication 1, dans lequel ledit solvant organique miscible à l'eau à l'étape (b) est choisi parmi un alcool, le tétrahydrofuranne et l'acétonitrile.

3. Procédé selon la revendication 2, dans lequel ledit alcool est choisi parmi le méthanol, l'éthanol, le n-propanol, l'isopropanol, le n-butanol, l'alcool isobutylique, le butanol tertiaire.

4. Procédé selon la revendication 1, dans lequel ledit- solvant non miscible à l'eau de l'étape (b) est choisi parmi l'acétate d'éthyle, la méthylisobutylcétone, le chloroforme, le dichlorométhane et le 1,2-dichloroéthane.

5. Procédé selon l'une quelconque des revendications précédentes, dans lequel la méthylisobutylcétone est employée dans un volume de 2,0 à 7,0 fois le poids du cefpodoxime proxétil impur.

6. Procédé selon l'une quelconque des revendications précédentes, dans lequel ledit halogénohydrate est choisi parmi l'acide chlorhydrique, l'acide bromhydrique et l'acide iodhydrique.

7. Procédé selon la revendication 6, dans lequel le rapport molaire de l'halogénure d'hydrogène utilisé est de 1,0 à 1,5 fois le cefpodoxime proxétil.

8. Procédé selon la revendication 1, dans lequel le sel d'halogénohydrate est isolé par filtration.

9. Procédé selon l'une quelconque des revendications précédentes, dans lequel ladite base est une base inorganique.

10. Procédé selon la revendication 9, dans lequel ladite base inorganique est choisie parmi le bicarbonate de sodium, l'hydroxyde de sodium, le carbonate de sodium, le carbonate de potassium et le bicarbonate de potassium.

11. Procédé selon l'une _quelconque des revendications précédentes, dans lequel le cefpodoxime proxétil pur est isolé par filtration.

12. Procédé selon l'une quelconque des revendications précédentes, dans lequel ledit cefpodoxime proxétil pur a un rapport diastéréomérique de 0,50 à 0,60.

13. Procédé selon l'une quelconque des revendications précédentes, dans lequel ledit traitement du sel d'halogénohydrate de cefpodoxime avec ladite base est effectué en 15 à 45 minutes, de préférence en 30 minutes.

14. Procédé selon la revendication 1 ou 13, dans lequel ledit traitement avec ladite base est effectué à une température de 15 à 40 °C, de préférence de 25 à 30 °C.

15. Procédé selon l'une quelconque des revendications précédentes, dans lequel, après ledit traitement avec ladite base, ledit mélange réactionnel est agité pendant 60 minutes.
